(19) Europäisches Patentamt
European Patent Office
Office européen des brevets

(11) Publication number: 0 199 131
A2

(12) **EUROPEAN PATENT APPLICATION**

(21) Application number: 86104119.2

(22) Date of filing: 25.03.86

(51) Int. Cl.[4]: C07C 69/704, C11D 1/08, C07C 67/08, A61K 7/50

(30) Priority: 26.03.85 IT 2007885

(43) Date of publication of application: 29.10.86 Bulletin 86/44

(84) Designated Contracting States: AT BE CH DE FR GB LI LU NL SE

(71) Applicant: Raffineria Olii Lubrificanti "R.O.L." S.p.A.
31, Foro Buonaparte
Milan(IT)

(72) Inventor: Turchini, Luigi
85, via Archimede
I-20129 Milan(IT)
Inventor: Garlisi, Salvatore, Dr.
32, via Aosta
I-13100 Vercelli(IT)
Inventor: Albanini, Aurelio, Dr.
37, via Vigorelli
I-27045 Casteggio Pavia(IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

(54) **Surfactants derived from citric acid.**

(57) Surfactants derived from citric acid having the general formula:

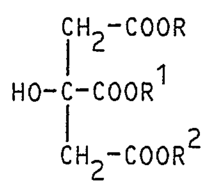

(I)

$-A_n - R^3$ (II)

wherein:

R, R', R² are equal or different from each other and are H, an alkaline metal, alkaline-earth metal or -NH4 cation, a cation of an organic ammonium base or a group having the formula:

$-A_n-R^3$ (II)

wherein:

A is a $C_2$-$C_4$ oxyalkylene group, n is a number ranging from 1 to 20 and $R^3$ is a $C_8$-$C_{20}$ alkyl group, at least one of said R, R', R² being an $-A_n-R^3$ group,

EP 0 199 131 A2

Rank Xerox

and wherein the -OH group of the compound of formula (I) can be esterified or etherified.

# SURFACTANTS DERIVED FROM CITRIC ACID

The present invention relates to non-toxic and biodegradable surfactants derived from citric acid.

In the large number of classes of known surfactants there is hardly a simple product possessing a combination of valuable properties such as biodegradability, non-toxicity, lack of skin-irritating properties, high solubility in water, together with excellent detergent properties, that would make such a product particularly versatile and, therefore, utilizable, in the same way, in the most different fields of use such as, for instance, in the field of detergents in general, in the field of toiletry, for beauty care, in the food and textile industry, in emulsion polymerization etc.

It has now been found that surfactants derived from citric acid, more particularly, surfactants consisting of esters of citric acid with aliphatic polyoxyalkylated alcohols, as well as of the salts thereof with inorganic or organic bases, show the above-mentioned characteristics in combination and, therefore, are useful in a wide field of application.

In accordance with the present invention, there are provided esters of citric acid having the general formula:

```
     CH2-COOR
     |
HO - C-COOR1        (I)
     |
     CH2-COOR2
```

wherein:

R, $R^1$, $R^2$ are equal or different from each other and represent a hydrogen atom, an alkaline metal, alkaline-earth metal or ammonium cation, a cation of an organic ammonium base or a group having the formula:

$-A_n-R^3$ (II)

wherein:

A is a $C_2$-$C_4$ oxyalkylene group, n is a number ranging between 1 and 20 and $R_3$ is a $C_8$-$C_{20}$ alkyl group, with the proviso that at least one of R, $R^1$, $R^2$ is an $-A_n-R^3$ group, and wherein the -OH group of the ester of formula (I) can be esterified or etherified according to usual methods.

The alkaline metal is preferably selected from sodium and potassium and the alkaline-earth metal is preferably magnesium. The cation of an organic ammonium base may, for instance, be derived from an alkanolamine such as triethanolamine. $R^3$ is preferably selected from linear or branched alkyl groups containing from 10 tó 16 carbon atoms. The oxyalkylene group A is preferably an oxyethylene group $-CH_2-CH_2O-$and n preferably ranges from 4 to 8.

The esters of formula (I) may be prepared by a process which comprises esterification of citric acid with an aliphatic polyoxyalkylated alcohol having the formula:

$R^3-A_n-OH$ (III)

wherein:

$R^3$, A and n are defined as above, by heating at a temperature of from 150 to 190°C, with continuous distillation of the water formed during the reaction and, optionally, converting the obtained product into a salt by means of an alkaline, alkaline-earth metal or ammonium base or an amine.

In the above-mentioned process, mono-, di-and tri-esters are obtained having the following formulae, respectively:

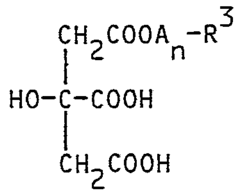 and/or 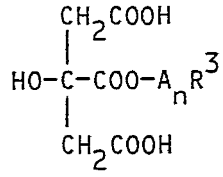 (IV)

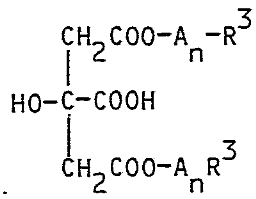 and/or 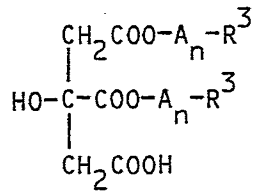 (V)

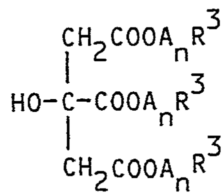 (VI)

Depending on reaction conditions employed and, in particular, on the molar ratios of citric acid to polyoxyalkylated alcohol, either monodi-or triesters are preferably formed.

In order to preferentially produce the monoester of formula (IV), citric acid and polyoxyalkylated alcohol are reacted in substantially equimolar ratio, whereas they are reacted in a molar ratio of about 1:2 in order to produce the diester of formula (V). Higher molar ratios lead to the formation of substantial amounts of the triester of formula (VI).

In the case of the preparation of the diester by using a polyoxyethylated alcohol, the reaction may be represented by the following reaction scheme:

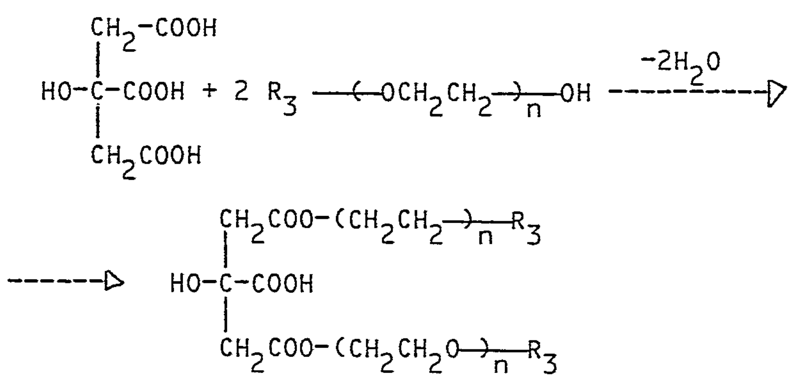


The polyoxyalkylated alcohols of formula (III) are known products and are commercially available. The ethoxylated alcohols may, for instance, be prepared by reacting the alcohol with ethylene oxide, using an alkaline base as a catalyst.

Preferred among the ethoxylated alcohols are those having the formula:

$R_3$-$(OCH_2CH_2)_n$-OH

wherein $R_3$ is a linear or branched alkyl group containing 12 to 16 carbon atoms and n ranges between 4 and 8, as well as the mixtures thereof.

The esters according to the present invention and, in particular, the monoesters and diesters of ethoxylated alcohols, their salts or mixtures thereof are very efficient surfactants, which allow a con siderable reduction of the surface tension even when used in a very low concentration and, therefore, may be used as emulsifying agents, dispersants and as detergents in general.

Besides the excellent detergent properties, they do not exhibit any toxic or skin-or eye-irritation effect and they do not have any acute toxicity, if ingested orally. They are highly biodegradable, the biodegradability being over 90 %. They are stable within a wide temperature range up to 100°C and after storage for a long time at low temperatures, they do not give rise to any separation when brought to room temperature. They show a good, but not an excessive wetting power and a good foamability. Their solubility in water ranges from moderate to excellent. In particular, their solubility increases with the increase of the number of moles of ethylene oxide contained in the alkylether chain. For instance, when the number of moles of ethylene oxide equals 3, the ester is not very soluble, whereas when the number of moles of ethylene oxide equals 7, the ester has good solubility.

The esters according to the invention are compatible with most known surfactants and, therefore, may be formulated with them. Because of their overall characteristics the esters according to the invention are very flexible in the different fields of application for surface-active agents. Owing to their high detergent power, combined with the lack of detrimental effects on the skin, the hair, the eyes, they are particularly suitable for beauty care applications, for instance, for the preparation of liquid or creamy detergents for the skin, shampoos and bath-foams.

The following examples will illustrate the invention, without limiting, however, its scope.

EXAMPLE 1

Esterification of citric acid

173.88 g (0.9 moles) of citric acid and 913.54 g (1.809 moles) of LIAL 123 ®.7 ETO (a mixture of ethoxylated alcohols having the formula

$$R^3 - [-OCH_2CH_2-]_n - OH,$$

wherein $R^3$ = $C_{11}$, $C_{12}$, $C_{13}$ and n = 7 with an average molecular weight of 505) were introduced, under nitrogen, into a reactor equipped with a heating system, stirrer, thermometer and a system for feeding the reactants and connected with a cooler provided with a manifold for collecting the reaction water.

While keeping the mixture under stirring and under a nitrogen flow, the temperature was brought to 180°C within about 90 minutes and kept at this value until the reaction water had distilled off almost completely (about 32-33 g). Then, the mixture was cooled to a temperature of 70°C and discharged from the reactor.

1053.6 g of a liquid product were obtained which had a water content below 0.1 %, an acid number of 59.0 and a saponification number of 126.3. The analysed product mainly consisted of the citric acid diester.

Salification

240 g of the citric acid ester prepared above and 726.6 g of demineralized water were introduced into a vessel equipped with stirrer, thermometer, dropping funnel and water cooling system.

The mixture was stirred until a homogeneous emulsion was obtained and then, under stirring, 33.4 g of a 30 % solution of NaOH were introduced slowly within about 1 hour through the dropping funnel, while keeping the temperature at values below 30°C.

1000 g of a limpid aqueous solution were obtained which contained 25 % by weight of citric ester sodium salt. The solution obtained could be used either as such or after dilution in the different fields of application for detergents.

After removal of the water by heating the solution at 50°C for 16 hours under vacuum, a product was obtained having a creamy consistency, which mainly consisted of the sodium salt of citric diester having an acid nubmer of 4.4, a saponification number of 66, an ester number of 61.6 and a pH of 7 at 1 %.

The salified product was perfectly soluble in water at any ratio, giving at 25°C limpid solutions up to a concentration of 25 % and more or less viscous solutions at higher concentrations

Furthermore, the following tests were carried out on the salified product:

-Surface tension

The surface tension measured at 20°C according to the Du NOÜY method was 31.5 dynes/cm at a concentration of 0.25 g/l and 31.0 dynes/cm at a concentration of 1 g/l.

-Biodegradability

The biodegradability was 96.9 % measured according to the method reported in EEC Directive 82/242.

-Acute oral toxicity

This was tested on Wistar rats, using the product at a concentration of 25 % in water according to the method of Thomson and Weil (Biometrics 8, 51, 1952). The results evaluated according to the sixth modification of EEC Directive 67/548 gave a $LD_{50}$>10.700 mg/kg both on the male and on the female. Therefore, the product proved to be non-toxic and not even noxious.

-Acute eye-irritation

The irritating effect on the eyes was evaluated on "New Zealand" white rabbits according to the Draize method (J.Pharmacol.Exp.Ther. 82,377-390, 1944). The global evaluation of the results was carried out according to the proposals of the FDA - (1965) as well as with reference to the standards and methods defined in EEC Directives 83/467 and 84/449. The product, at a concentration of 25 % in water, turned out to be non-irritating. Sensible and persistent impairments were observed neither on the cornea, nor on the iris and not even on the conjunctiva.

-Primary dermal irritation

The test was carried out on "New Zealand" white rabbits, using the product at a concentration of 25 % in water and applying it on uninjured skin and on scarified skin over 24 hours. The results were evaluated according to the FDA rules, the OECD rules (Final Report: Acute Dermal Irritation and Corrosion 1981) and the AFNOR (1982) rules referring to the standards and methods as defined in EEC Directives 83/467 and 84/449.

The product turned out to be non-irritant as neither oedema nor erythema together with eschar were noticed both on uninjured skin and on scarified skin. Furthermore, the salified product showed an excellent detergent power comparable with that of the best surfactants used in the detergency field such as, for instance, laurylether sul-phates, dodecylbenzene sulphonates and ethoxylated fatty alcohols and nonylphenols.

EXAMPLE 2

Esterification of citric acid

288.2 g (1.5 moles) of citric acid and 761.3 - (1.507 moles) of LIAL 123 ®.7ETO (a mixture of ethoxylated alcohols having the formula

$$R^3-[-OCH_2CH_2-]_n-OH,$$

, wherein $R^3$ = $C_{11}$, $C_{12}$,$C_{13}$ and n = 7, with an average molecular weight of 505) were introduced, under nitrogen, into a reactor equipped with a heating system, stirrer, thermometer and a system for feeding the reactants and connected with a cooler provided with a manifold for collecting the reaction water.

While keeping the mixture under stirring and under a nitrogen flow, the temperature was brought to 155°C within about 80 minutes and kept at this value until the reaction water had distilled off almost completely (about 27 g). Then, the mixture was cooled to a temperature of 80°C and discharged from the reactor.

1049 g of a liquid product were obtained having a water content below 0.1 %, an acid number of 156.6 and a saponification number of 231.5. The analysed product mainly consisted of citric acid monoester.

Salification

225 g of the citric ester prepared above and 691.2 g of demineralized water were introduced into a vessel equipped with stirrer, thermometer, dropping funnel and water cooling system.

The mixture was stirred until a homogeneous emulsion was obtained and then, under stirring, 83.8 g of a 30 % solution of NaOH were introduced slowly within 70 minutes through the dropping funnel, while keeping the temperature at values below 30°C

1000 g of a limpid aqueous solution were obtained which contained 24.9 % by weight of citric ester sodium salt.

The solution thus obtained could be used either as such or after dilution in the different fields of application of detergents.

After removal of the water by heating the solution at 50°C for 16 hours under vacuum, a product was obtained having a creamy consistency and mainly consisting of the sodium salt of citric monoester having an acid number of 6, a saponification number of 67.2, an ester number of 61.2 and a pH of 7 at 1 %.

The salified product dissolved in water at any ratio and, subjected to the same tests as in example 1, showed properties similar to those of the salified product of example 1.

EXAMPLE 3

A shampoo was prepared having the following composition (by weight):

68% of demineralized water,

28 % of the product obtained in example 1 at a concentration of 25 % in water,

2 % of diethanolamide of coconut fatty acids,

2 % of polyethylene glycol distearate (M.W. = 6000).

The first three ingredients were mixed and heated at 60°C, whereafter the fourth ingredient was added and the mixture was stirred at this temperature until complete dissolution.

EXAMPLE 4

A bath foam was prepared having the following composition (by weight):

40% of demineralized water,

56 % of the product obtained in example 1 at a concentration of 25 % in water,

2 % of diethanolamide of coconut fatty acids,

2 % of polyethylene glycol distearate (M.W. = 6000).

The foam was prepared under the same conditions as described in example 3.

EXAMPLE 5

Esterification of citric acid

173.88 g (0.9 moles) of citric acid and 913.55 g (1.809 moles) of LIAL 123 ®.7ETO (a mixture of ethoxylated alcohols having the formula

$$R^3 - [-OCH_2-CH_2^2-]_n - OH,$$

wherein $R^3$ = $C_{11}$, $C_{12}$, $C_{13}$ and n = 7, with an average molecular weight of 505) were introduced, under nitrogen, into a reactor equipped with stirrer, thermometer and a system for feeding the reactants and connected with a cooler provided with a manifold for collecting the reaction water.

While keeping the mixture under stirring and under a nitrogen flow, the temperature was brought to 178-180°C within about 80 minutes and kept at this value until the reaction water had distilled off almost completely (about 32.4 g). The mixture was then cooled to a temperature of 80°C and discharged from the reactor.

1053.6 g of a liquid product were obtained having a water content of about 0.1 %, an acid number of 59.0 and a saponification number of 126.3. The analysed product mainly consisted of citric acid diester.

Salification

736.4 g of demineralized water and 6.6 g of Mg(OH)2 were introduced into a vessel equipped with stirrer, thermometer, dropping funnel and water cooling system. The mixture was stirred for a few minutes at a temperature of 20-22°C until a

milky dispersion was obtained and then, always under stirring, 257.0 g of the citric ester prepared above were introduced slowly within about 5 hours through the dropping funnel, while keeping the temperature at values not higher than 24°C and continuing with stirring for 30 minutes, when the addition was completed.

1000 g of a limpid solution were obtained containing 26.3 % by weight of citric diester magnesium salt.

The solution obtained could be used either as such or after dilution in the different fields of application of detergents.

By removal of the water from the solution a product was obtained having a creamy consistency, which mainly consisted of the magnesium salt of citric diester having an acid number of 39.2, a saponification number of 131.9 and a pH of 6.1 at 1 %.

The salified product dissolved in water at any ratio and, subjected to the same tests described in example 1, showed characteristics similar to those of the salified product of example 1.

EXAMPLE 6

Esterification of citric acid

172.8 g (0.9 moles) of citric acid and 732.4 g - (1.356 moles) of LOROL/V®.7.8ETO (a mixture of natural ethoxylated alcohols having the formula

$$R^3 - [-OCH_2CH_2-]_n - OH,$$

, wherein $R^3 = C_{12}, C_{14}, C_{16}$ and n = 7.8, with an average molecular weight of 540) were introduced, under a nitrogen flow, into a reactor equipped with a heating system, stirrer, thermometer and a system for feeding the reactants and connected with a cooler provided with a manifold for collecting the reaction water.

While keeping the mixture under stirring and under a nitrogen flow, the temperature was brought to 178-180°C within about 80 minutes and kept at this value until about 45 % of the theoretical reaction water (about 24.3 g) were distilled off.

At this point the course of the esterification was monitored by determining the acid number When the acid number reached a value of 88-91 - (theorectical value 86.3) the esterification had to be considered complete.

The mixture was then cooled to a temperature of 80°C and discharged from the reactor.

880 g of a limpid liquid product were obtained having a water content of about 0.1 %, an acid number of 89.8 and a saponification number of 156.4. The analysed product mainly consisted of a mixture of citric acid mono-and diesters.

Salification

234.7 g of the mixture of citric acid mono-and diesters prepared above and 714.8 g of demineralized water were introduced into a vessel equipped with stirrer, thermometer, dropping funnel and a water cooling system. The mixture was heated, under stirring, at 45-50°C until the gels disappeared and a homogeneous emulsion was formed. After cooling down to 20-22°C the evaporated water was replenished and then, under stirring, 50.5 g of 30 % NaOH were introduced slowly through the dropping funnel within about 70 minutes, while keeping the temperature at values not over 24°C.

1000 g of a limpid solution were obtained containing 25 % by weight of a mixture of citric acid mono-and diester sodium salts.

By removal of water from the solution a product was obtained having a creamy consistency, which mainly consisted of the sodium salt of citric mono-and diester having an acid number of 4.4, a saponification number of 58 and a pH of 6.9 at 1 %.

The salified product dissolved in water of any ratio and, subjected to the tests described in example 1, showed characteristics similar to those of the salified product of example 1.

EXAMPLE 7

A shampoo was prepared having the following composition (by weight):

30.7 % of demineralized water,

52.0 % of the product obtained in example 1 at a concentration of 25 % in water,

14.3 % of Na oleylsarkosinate at 21 %,

2 % of diethanolamide of coconut fatty acid,

1 % of polyethylene glycol distearate (M.W. = 6000).

The first four ingredients were mixed and heated at 60°C, whereafter the fifth ingredient was added and the mixture was stirred at this temperature until complete dissolution.

The shampoo obtained was a limpid liquid at 20°C, having a viscosity of 1092 cst and containing 16 % of active component.

Its foam-forming ability determined on 200 ml of an aqueous solution containing 2 g/l of the product by using a plunger system with a bored disk (50 strokes) was as follows:

| After minutes | ml of foam |
|---|---|
| 0 | 780 |
| 5 | 770 |
| 10 | 770 |
| 15 | 760 |
| 20 | 760 |
| 25 | 750 |
| 30 | 730 |

EXAMPLE 8

A liquid detergent for kitchenware was prepared by mixing the following ingredients:

| | % by weight |
|---|---|
| Demineralized water | 40.95 |
| Product obtained in example 1 at a concentration of 25 % in $H_2O$ | 52 |
| Diethanolamide of coconut fatty acids | 1.0 |
| LIAL 123®.9.9ETO | 4.0 |
| Sorbitan monolaurate | 0.8 |
| Essence of lemon | 0.2 |
| Yellow dye | 0.002 |
| Preserving agent PARMETOL K-40® | 0.10 |
| EDTA sodium salt | 0.15 |
| Fluorescein sodium salt | 0.0006 |
| Polyethylene glycol distearate (M.W.6000) | 0.8 |

The obtained detergent,at 20°C, was a limpid liquid having a viscosity of 242 cst and containing 17 % of active component.

Its foam-forming activity, determined as in example 7, was as follows:

| After minutes | ml of foam |
|---|---|
| 0 | 630 |
| 5 | 610 |
| 10 | 590 |
| 15 | 520 |
| 20 | 440 |
| 25 | 400 |
| 30 | 350 |

**Claims**

1. Citric acid esters having the general formula

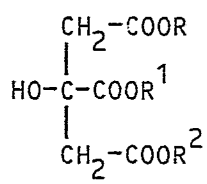

(I)

wherein:

R, R', $R^2$ are equal or different from each other and represent a hydrogen atom, an alkaline metal,alkaline-earth metal or ammonium cation, a cation of an organic ammonium base, or a group having the formula:

$-A_n-R^3$ (II)

wherein:

A is a $C_2$-$C_4$ oxyalkylene group, n is a number ranging between 1 and 20 and $R^3$ is a $C_8$-$C_{20}$ alkyl group, with the proviso that at least one of R, R', $R^2$ is an $-A_n-R^3$ group.

2. Esters according to claim 1, wherein in group $-A_n-R^3$ (II), A is an oxyethylene group $-CH_2CH_2O-$, n is a number ranging between 4 and 8, and $R^3$ is an alkyl group containing from 10 to 16 carbon atoms.

3. Esters according to claim 1, wherein R is $-A_n-R^3$ and R' and $R^2$ are sodium, potassium, magnesium or ammonium cations or wherein R' is $-A_n-R^3$ and R and $R^2$ are sodium, potassium, magnesium or ammonium cations.

4. Esters according to claim 1, wherein R and R' are $-A_n-R^3$ and $R^2$ is a sodium, potassium, magnesium or ammonium cation or R and $R^2$ are $-A_n-R^3$ and R' is a sodium, potassium, magnesium or ammonium cation.

5. Esters according to claim 1, wherein the -OH group of the ester of formula (I) has been esterified or etherified according to conventional methods.

6. A process for the preparation of esters having the formula (I) according to claim 1, which comprises esterifying citric acid with an aliphatic polyoxyalkylated alcohol having the formula:

$R^3 -A_n -OH$ (III)

wherein:

$R^3$, A and n are defined as in claim 1, at a temperature of from 150 to 190°C, with continuous removal of the reaction water, and optionally converting the obtained product into a salt by means of an alkaline, alkaline-earth or ammonium base or an amine.

7. A process according to claim 6, wherein in the alcohol of formula (III),A is an oxyethylene group $-CH_2CH_2O-$, n is a number ranging between 4 and 8, and $R^3$ is an alkyl group containing from 10 to 16 carbon atoms.

8. A process according to claim 6 or 7, wherein the base is selected from sodium, potassium, magnesium or ammonium hydroxide, sodium or potassium carbonate or bicarbonate and triethanolamine.

9. Cosmetic and detergent compósitions containing one or more citric acid esters of formula (I) according to any one of claims 1 to 5.

10. Use of the citric acid esters of formula (I) according to any one of claims 1 to 5 as surface-active agents.